# EUROPÄISCHE PATENTSCHRIFT

(11) **EP 0 599 148 B1**
(45) Veröffentlichungstag und Bekanntmachung des Hinweises auf die Patenterteilung: **12.03.1997**
(21) Anmeldenummer: 93118285.1
(22) Anmeldetag: 11.11.1993
(51) Int. Cl.: C07C 45/49, C07C 47/565, C07C 47/57

(54) **Verfahren zur selektiven Herstellung von Hydroxybenzaldehyden**
Process for the selective preparation of hydroxybenzaldehydes
Procédé pour la préparation sélective d'hydroxybenzaldéhydes

(30) Priorität: 20.11.1992 DE 4239101
(43) Veröffentlichungstag der Anmeldung: 01.06.1994
(73) Patentinhaber: HOECHST AKTIENGESELLSCHAFT, 65926 Frankfurt am Main (DE)
(72) Erfinder: Weisse, Laurent, Dr., D-61440 Oberursel (DE); Neunteufel, Robert, Dr., D-31542 Bad Nenndorf (DE); Strutz, Heinz, Dr., D-61250 Usingen (DE)

(56) Entgegenhaltungen:
- US-A- 2 485 237
- US-A- 3 098 875
- CHEMICAL ABSTRACTS, vol. 070, no. 7, 17. Februar 1969, Columbus, Ohio, US; abstract no. 028557, KUDO K ET AL 'Reaction of phenol with carbon monoxide under high pressure in the medium of hydrogen fluoride and boron fluoride'

## Beschreibung

Die vorliegende Erfindung betrifft ein verbessertes, technisch einfaches Verfahren zur Herstellung von Hydroxybenzaldehyden durch selektive Formylierung von Phenol und Phenolderivaten zu den entsprechenden Hydroxybenzaldehyden.

Hydroxybenzaldehyde sind wichtige Zwischenprodukte für Zimtsäurederivate, die als UV-Absorbenzien in der Kosmetikindustrie eingesetzt werden, ferner für optische Aufheller, Farbstoffe, Pharma- und Pflanzenschutzprodukte (DE-OS 27 32 227). 3,5-Dialkyl-4-hydroxybenzaldehyde werden als Antioxidationsmittel eingesetzt (US 3 974 223). Sie sind außerdem wichtige Ausgangsmaterialien bei der Synthese von Benzoxazinen, eine Verbindungsklasse, die insbesondere im Bereich Pflanzenschutz technisches Interesse findet (EP 289 171). Ferner sind sie wichtige Zwischenprodukte für industrielle Synthesen von Riechstoffen und Fotochemikalien (EP 451 650).

Zur Herstellung der Hydroxybenzaldehyde ist eine Reihe von Verfahren bekannt, wie aus fast jedem Lehrbuch der organischen Chemie ersichtlich (Houben-Weyl E3, 4. Auflage). Alle diese Verfahren besitzen jedoch einen oder mehrere der folgenden Nachteile: Bildung von schlecht zu trennenden Isomeren, mehrstufige Synthese, hoher Salzballast, geringe Ausbeuten, schwer zugängliche Ausgangssubstanzen (DE 29 04 315, DE-OS 27 36 523).

Eine bekannte direkte Methode zur Herstellung von Hydroxybenzaldehyden ist die Carbonylierung der entsprechenden Phenole. Dabei zeigt sich jedoch, daß Phenole mit Hilfe der Gattermann-Koch-Reaktion nicht carbonyliert werden können (Advanced Organic Chemistry, 1983, McGraw-Hill, 494). Außerdem führt die Einsatzmenge an Aluminiumchlorid zu erheblichen Abwasserproblemen.

Die Formylierung von Phenolen mit Orthoameisensäureesterdichlorid bzw. Orthoameisensäureester und Aluminiumtrichlorid bzw. Titantetrachlorid im Überschuß ist wegen der schlechten Ausbeuten, der geringen Selektivität und der damit verbundenen Trennprobleme, sowie der mit dem Einsatz großer Mengen Metallhalogenide einhergehenden Abwasserproblematik technisch nicht tragbar (Chem. Ber. 96 (1963) 308).

Die Verwendung eines großen Überschusses an Urotropin führt bei der Duff-Reaktion auch zu Abwasserbelastungen (BE 841 523, DE 33 04 202). Außerdem sind die Ausbeuten meist gering (Advanced Organic Chemistry, 1983, McGraw-Hill, Tokyo, 496).

In DE-OS 27 32 227 ist die Gattermann-Reaktion mit Cyanwasserstoff in Fluorwasserstoff beschrieben, die jedoch zu erheblichen sicherheitstechnischen Problemen führt.

Es ist ferner bekannt, daß Phenolderivate, wie Guajacol, in einem Fluorwasserstoff/Bortrifluorid-Gemisch mit Methylformiat carbonyliert werden können (EP 300 861). Diese Reaktion führt aber meistens zu Produktgemischen und benötigt einen sehr großen Überschuß an Bortrifluorid.

Es ist außerdem bekannt, daß Phenol mit Kohlenmonoxid in Fluorwasserstoff/Bortrifluorid zu p-Hydroxybenzaldehyd umgesetzt werden kann (Sekiyu Gakkai Shi 11(1968)690). Zum vollständigen Umsatz wird nach dieser Methode ein Kohlenmonoxiddruck von 300 bar benötigt, wobei 61 bzw. 13 Molprozent para-bzw. ortho-Hydroxybenzaldehyd, d.h. ein Isomerengemisch, gebildet werden.

Die US-PS 2 485 237 betrifft ein Verfahren zur Herstellung von aromatischen Aldehyden durch Umsetzung eines aromatischen Kohlenwasserstoffs mit Kohlenmonoxid in Gegenwart eines HF/BF₃-Katalysators. In Beispiel 3 beträgt bei einem Toluolumsatz von 80,5% die Selektivität 92%. Daraus resultiert eine Ausbeute an Tolylaldehyd von 74%. Eine Umsetzung eines Phenols ist nicht erwähnt. Die Umsetzung erfolgt in allen Beispielen in einem Temperaturintervall.

Es bestand somit ein Bedürfnis nach einem selektiven Verfahren zur Herstellung von Hydroxybenzaldehyden, durch das die den bekannten Verfahren innewohnenden Nachteile vermieden werden.

Es wurde nun überraschenderweise gefunden, daß man Hydroxybenzaldehyde der allgemeinen Formel (1) 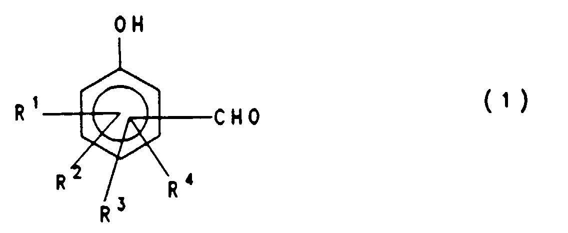 in welcher R¹, R², R³ und R⁴ gleich oder verschieden sind, Wasserstoff-, Fluor-, Chlor- oder Bromatome, ferner Alkyl(C₁-C₆)-gruppen, die durch Fluoratome substituiert sein können, Alkoxy(C₁-C₄)-gruppen, Aryl(C₆-C₁₄)-gruppen, wie beispielsweise Phenyl- oder Naphthylgruppen, die am aromatischen Kern durch Fluor-, Chlor- oder Bromatome, Alkyl(C₁-C₄)- oder Alkoxy(C₁-C₄)-gruppen substituiert sein können, ferner Aryl(C₆-C₁₄)-alkyl(C₁-C₆)-gruppen, wie beispielsweise Phenyl-alkyl(C₁-C₆)- oder Naphthyl-alkyl(C₁-C₆)-gruppen, die am Arylrest durch Fluor-, Chlor- oder Bromatome, Alkyl(C₁-C₄)- oder Alkoxy(C₁-C₄)-gruppen substituiert sein können, ferner den Rest eines fünf- oder sechsgliedrigen, gesättigten oder ungesättigten Carbocyclus, beispielsweise eine Cyclopentyl-, Cyclopentadienyl-, Cyclohexyl-, Cyclohexenyl- oder Cyclohexadienylgruppe, der durch Alkyl(C₁-C₄)-gruppen substituiert sein kann, ferner Aryl(C₆-C₁₀)-oxy-gruppen, beispielsweise die Phenoxygruppe, die am aromatischen Kern durch Fluor-, Chlor- oder Bromatome, Alkyl(C₁-C₄)- oder Alkoxy(C₁-C₄)-gruppen substituiert sein können, bedeuten und wobei die Substituenten R¹, R², R³ und R⁴ mit den Kohlenstoffatomen des Hydroxybenzolrings, an denen sie sich befinden, einen oder zwei gesättigte oder ungesättigte isocyclische oder heterocyclische Ringe bilden können, die beispielsweise durch Alkyl(C₁-C₄)-gruppen, substituiert sein können, und in welcher die Hydroxy- und Aldehydgruppen in ortho- oder para-Stellung zueinander stehen, in vorteilhafter Weise herstellen kann, indem man 1 Mol eines Phenols der allgemeinen Formel (2) 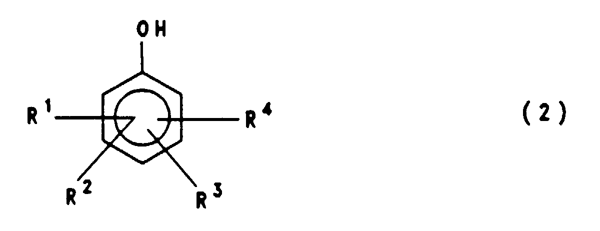 in welcher R¹, R², R³ und R⁴ die vorstehend genannten Bedeutungen haben, gegebenenfalls in einem gegenüber den Reaktionsteilnehmern inerten organischen Lösungsmittel, in einem Druckgefäß mit 5 bis 100 Mol, vorzugsweise 20 bis 50 Mol Fluorwasserstoff und 0,5 + x bis 1,5 + x Mol, vorzugsweise 0,9 + x bis 1,2 + x Mol, wobei x die Zahl der in der Ausgangsverbindung der genannten Formel (2) enthaltenen Sauerstoffatome bedeutet, besonders bevorzugt mit 1 Mol Bortrifluorid versetzt, die Temperatur dieser Mischung auf -10° bis 100°C, vorzugsweise 0° bis 80°C, stellt und anschließend in diese vorgelegte Mischung Kohlenmonoxid einleitet bis ein Druck von 10 bis 150 bar, vorzugsweise 20 bis 100 bar, erreicht ist und bei dem erreichten gewünschten Druck reagieren läßt.

Zum erfindungsgemäßen Verfahren sei im einzelnen noch folgendes ausgeführt:

Gegenüber den Reaktionsteilnehmern inerte organische Lösungsmittel sind beispielsweise Methylenchlorid, Chloroform, Tetrachlorkohlenstoff, Dichlorethan, Hexan und Heptan. Bevorzugt wird jedoch mit reinem, wasserfreien Fluorwasserstoff, d.h. in Abwesenheit eines inerten Lösungsmittels, umgesetzt.

Die Reaktionszeiten schwanken in der Regel zwischen 30 Minuten und 50 Stunden, vorzugsweise zwischen 1 Stunde und 24 Stunden.

Bevorzugt wird die Verbindung der genannten allgemeinen Formel (2) vorgelegt und der Fluorwasserstoff zudosiert, jedoch ist auch die umgekehrte Zugabefolge möglich. Anschließend wird das Bortrifluorid zweckmäßigerweise auf einmal zugegeben. Die Zugabe kann jedoch auch schrittweise erfolgen. Nach Erreichen der gewünschten Reaktionstemperatur wird der gewünschte Kohlenmonoxiddruck eingestellt und während der Reaktion, falls erforderlich, nachreguliert. Der Kohlenmonoxiddruck kann aber auch so eingestellt werden, daß er während der Umsetzung nicht nachjustiert werden muß.

Die jeweils optimalen Temperaturen innerhalb des weiter oben genannten allgemeinen und bevorzugten Temperaturbereichs sind in Abhängigkeit der jeweils eingesetzten Ausgangsverbindung der genannten Formel (2), d.h. in Abhängigkeit von den am Benzolkern der Ausgangsverbindung befindlichen Substituenten, zu wählen und anzuwenden.

Das erfindungsgemäße Verfahren zeichnet sich besonders dadurch aus, daß unterschiedlich substituierte Hydroxybenzaldehyde in einer einfachen und kurzen Synthese (Einstufenprozeß) sehr selektiv und in bis zu quantitativen Ausbeuten erhalten werden können. Es weist gegenüber den zum Stand der Technik zählenden Verfahren den Vorteil auf, auch bei relativ niedrigem Druck hohe Umsätze und Selektivitäten zu erzielen. Von Vorteil ist weiterhin, daß es grundsätzlich möglich ist, den als Katalysator wirkenden Fluorwasserstoff bzw. das Bortrifluorid zurückzugewinnen.

Die nachstehenden Beispiele dienen zur näheren Erläuterung des erfindungsgemäßen Verfahrens.

### Beispiel 1

### Herstellung von 4-Hydroxybenzaldehyd

9,41 g (100 mmol) Phenol werden in einem 250 ml Edelstahlautoklav mit 100 g (5 mol) wasserfreiem Fluorwasserstoff und 14 g (206 mmol) Bortrifluorid versetzt. Anschließend wird das Reaktionsgemisch auf 40°C erwärmt und Kohlenmonoxid solange eingeleitet, bis der Druck 50 bar erreicht hat. Dieser Druck muß innerhalb der Reaktionszeit mehrmals nachreguliert werden. Nach einer Stunde wird die Reaktionslösung auf 1 Kilo Eis gegossen und mit konzentrierter KOH-Lösung neutralisiert. Nach mehrmaligem Extrahieren der wäßrigen Phase mit Essigester werden die abgetrennten und vereinigten organischen Phasen mit MgSO₄ getrocknet. Unter vermindertem Druck wird das Lösemittel abdestilliert, wobei 12,2 g eines Feststoffs isoliert werden. Nach einem quantitativen ¹H-NMR-Spektrum wird der Gehalt an 4-Hydroxybenzaldehyd zu 80 % bestimmt (80 % d.Th.), wobei der Anteil an Salicylaldehyd geringer als 0,1 % ist.

### Beispiel 2

### Herstellung von 4-Hydroxy-3-methylbenzaldehyd

10,8 g (100 mmol) o-Kresol werden in einem 250 ml Edelstahlautoklav mit 100 g (5 mol) wasserfreiem Fluorwasserstoff und 13,8 g (203 mmol) Bortrifluorid versetzt. Anschließend wird das Reaktionsgemisch auf 40°C erwärmt und Kohlenmonoxid solange eingeleitet bis der Druck 100 bar erreicht hat. Dieser Druck muß innerhalb der Reaktionszeit mehrmals nachreguliert werden. Nach 14 Stunden wird die Reaktionslösung auf 1 Kilo Eis gegossen und mit konzentrierter KOH-Lösung neutralisiert. Nach der weiteren Aufarbeitung gemäß Beispiel 1 werden 13,5 g eines Öls isoliert. Anhand des ¹H-NMR-Spektrums und des Gaschromatogramms wird die Reinheit des erhaltenen 4-Hydroxy-3-methylbenzaldehyds mit 95 % bestimmt (94 % d.Th.).

### Beispiel 3 (Vergleichsbeispiel)

### Herstellung von 4-Hydroxy-3-methylbenzaldehyd

10,8 g (100 mmol) o-Kresol werden in einem 250 ml Edelstahlautoklav mit 100 g (5 mol) wasserfreiem Fluorwasserstoff und 14 g (206 mmol) Bortrifluorid versetzt. Anschließend wird zunächst bei Raumtemperatur in das Reaktionsgemisch solange Kohlenmonoxid eingeleitet bis der Druck 100 bar erreicht hat. Dann wird auf 40°C erwärmt und 22 Stunden gerührt. Darauf wird die Reaktionslösung auf 1 Kilo Eis gegossen und mit konzentrierter KOH-Lösung neutralisiert. Nach der weiteren Aufarbeitung gemäß Beispiel 1 werden 13,5 g eines Produktes isoliert. Das Molverhältnis von 4-Hydroxy-3-methylbenzaldehyd zum isomeren 2-Hydroxy-3-methylbenzaldehyd beträgt 83:17 (82 bzw. 17% d.Th.).

### Beispiel 4

### Herstellung von 4-Hydroxy-3,5-dimethylbenzaldehyd

12,2 g (100 mmol) 2,6-Dimethylphenol werden in einem 250 ml Edelstahlautoklav mit 100 g (5 mol) wasserfreiem Fluorwasserstoff und 14,5 g (213 mmol) Bortrifluorid versetzt. Anschließend wird in das Reaktionsgemisch bei 22°C Kohlenmonoxid solange eingeleitet bis der Druck 110 bar erreicht hat. Dieser Druck muß innerhalb der Reaktionszeit mehrmals nachreguliert werden. Nach einer Stunde wird die Reaktionslösung auf 1 Kilo Eis gegossen und mit konzentrierter KOH-Lösung neutralisiert. Nach der weiteren Aufarbeitung gemäß Beispiel 1 erhält man 14,8 g eines Feststoffs, der anhand des ¹H-NMR-Spektrums als 4-Hydroxy-3,5-dimethylbenzaldehyd charakterisiert wird. Die Reinheit dieser erhaltenen Verbindung beträgt 98 % (97 % d.Th.).

Legt man das 2,6-Dimethylphenol gelöst in Methylenchlorid, Chloroform oder Tetrachlorkohlenstoff vor und arbeitet im übrigen wie in diesem Beispiel beschrieben, so gelangt man praktisch zum gleichen Ergebnis.

### Beispiel 5

### Herstellung von 2-Hydroxy-4,5-dimethylbenzaldehyd

12,2 g (100 mmol) 3,4-Dimethylphenol werden in einem 250 ml Edelstahlautoklav mit 100 g (5 mol) wasserfreiem Fluorwasserstoff und 14,2 g (208 mmol) Bortrifluorid versetzt. Anschließend wird das Reaktionsgemisch auf 40°C erwärmt und Kohlenmonoxid solange eingeleitet, bis der Druck 110 bar erreicht hat. Dieser Druck muß innerhalb der Reaktionszeit mehrmals nachreguliert werden. Nach 22 Stunden wird die Reaktionslösung auf 1 Kilo Eis gegossen und mit konzentrierter KOH-Lösung neutralisiert. Nach der weiteren Aufarbeitung gemäß Beispiel 1 isoliert man 14,8 g 2-Hydroxy-4,5-dimethylbenzaldehyd, dessen Reinheit gaschromatographisch mit 98 % bestimmt wird (97 % d.Th.). Die Bildung des Isomeren der Hauptkomponente, 5,6-Dimethyl-2-hydroxybenzaldehyd, wird dabei nicht beobachtet.

### Beispiel 6 (Vergleichsbeispiel)

### Herstellung von 2-Hydroxy-4,5-dimethylbenzaldehyd

12,2 g (100 mmol) 3,4-Dimethylphenol werden in einem 250 ml Edelstahlautoklav mit 100 g (5 mol) wasserfreiem Fluorwasserstoff und 14 g (206 mmol) Bortrifluorid versetzt. Anschließend wird zunächst bei Raumtemperatur in das Reaktionsgemisch solange Kohlenmonoxid eingeleitet bis der Druck 100 bar erreicht hat. Dann wird auf 40°C erwärmt und 22 Stunden gerührt. Darauf wird die Reaktionslösung auf 1 Kilo Eis gegossen und mit konzentrierter KOH-Lösung neutralisiert. Nach der weiteren Aufarbeitung gemäß Beispiel 1 werden 13,5 g eines Feststoffs, bestehend aus 2-Hydroxy-4,5-dimethylbenzaldehyd und 5,6-Dimethyl-2-hydroxybenzaldehyd im Verhältnis 95:5 (85 bzw. 4,5 % d.Th.) erhalten.

### Beispiel 7

### Herstellung von 3-Hydroxy-5,6,7,8-tetrahydro-2-naphthaldehyd

5 g (33,7 mmol) 5,6,7,8-Tetrahydro-2-naphthol werden in einem 250 ml Edelstahlautoklav mit 50 g (2,5 mol) wasserfreiem Fluorwasserstoff und 5 g (73 mmol) Bortrifluorid versetzt. Anschließend wird das Reaktionsgemisch auf 40°C erwärmt und Kohlenmonoxid solange eingeleitet bis der Druck 150 bar erreicht hat. Dieser Druck muß innerhalb der Reaktionszeit mehrmals nachreguliert werden. Nach 18 Stunden wird die Reaktionslösung auf 1 Kilo Eis gegossen und mit konzentrierter KOH-Lösung neutralisiert. Nach der weiteren Aufarbeitung gemäß Beispiel 1 isoliert man 5,7 g eines Feststoffs, dessen ¹H-NMR-Spektrum mit einer Vergleichssubstanz von 5,6,7,8-tetrahydro-2-naphthaldehyd übereinstimmt. Die GC-Reinheit beträgt 95 % (91 % d.Th.).

Legt man das 5,6,7,8-Tetrahydro-2-naphthol gelöst in Dichlormethan, Hexan oder Heptan vor und verfährt im übrigen wie in diesem Beispiel beschrieben, so gelangt man praktisch zum gleichen Ergebnis.

### Beispiel 8

### Herstellung von 4-Hydroxy-3-ethylbenzaldehyd

12,2 g (100 mmol) 2-Ethyl-phenol werden in einem 250 ml Edelstahlautoklav mit 100 g (5 mol) wasserfreiem Fluorwasserstoff und 14,5 g (212 mmol) Bortrifluorid versetzt. Anschließend wird das Reaktionsgemisch auf 40°C erwärmt und Kohlenmonoxid solange eingeleitet bis der Druck 50 bar erreicht hat. Dieser Druck muß innerhalb der Reaktionszeit mehrmals nachreguliert werden. Nach 1 Stunde wird die Reaktionslösung auf 1 Kilo Eis gegossen und mit konzentrierter KOH-Lösung neutralisiert. Nach der weiteren Aufarbeitung gemäß Beispiel 1 werden 10,6 g eines braunen Öls isoliert, das als 4-Hydroxy-3-ethylbenzaldehyd charakterisiert wird. Die gaschromatographisch bestimmte Reinheit beträgt 95 % (91 % d.Th.).

## Patentansprüche

1. Verfahren zur Herstellung von Hydroxybenzaldehyden der allgemeinen Formel (1) 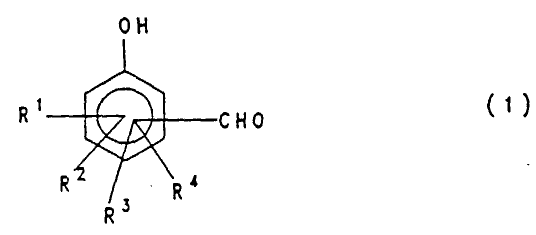 in welcher R¹, R², R³ und R⁴ gleich oder verschieden sind,
Wasserstoff-, Fluor-, Chlor- oder Bromatome, ferner Alkyl(C₁-C₆)-gruppen, die durch Fluoratome substituiert sein können,
Alkoxy(C₁-C₄)-gruppen, Phenyl- oder Naphthylgruppen, die am aromatischen Kern durch Fluor-, Chlor- oder Bromatome, Alkyl(C₁-C₄)- oder Alkoxy(C₁-C₄)gruppen substituiert sein können, ferner Phenyl-alkyl(C₁-C₆)- oder Naphthylalkyl(C₁-C₆)-gruppen, die am aromatischen Kern durch Fluor-, Chlor- oder Bromatome oder Alkyl(C₁-C₄)- oder Alkoxy(C₁-C₄)-gruppen substituiert sein können, ferner eine Cyclopentyl-, Cyclopentadienyl-, Cyclohexyl-, Cyclohexenyl- oder Cyclohexadienylgruppe, die durch Alkyl(C₁-C₄)-gruppen substituiert sein können, ferner die Phenoxygruppe, die durch Fluor-, Chlor- oder Bromatome, Alkyl(C₁-C₄)- oder Alkoxy(C₁-C₄)-gruppen substituiert sein kann, bedeuten und wobei die Substituenten R¹, R², R³ und R⁴ mit den Kohlenstoffatomen des Hydroxybenzolrings, an denen sie sich befinden, einen oder zwei gesättigte oder ungesättigte isocyclische oder heterocyclische Ringe bilden können, die durch Alkyl(C₁-C₄)-gruppen substituiert sein können, und in welcher die Hydroxyl- und Aldehydgruppen in ortho- oder para-Stellung zueinander stehen, dadurch gekennzeichnet, daß man 1 Mol eines Phenols der allgemeinen Formel (2) 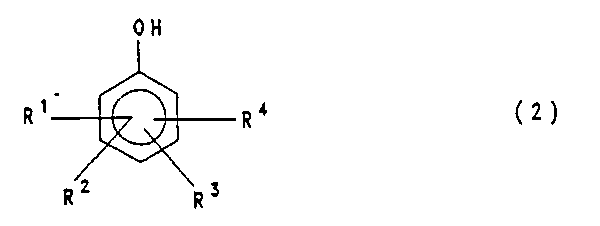 in welcher R¹, R², R³ und R⁴ die vorstehend genannten Bedeutungen haben, gegebenenfalls in einem gegenüber den Reaktionsteilnehmern inerten organischen Lösungsmittel, in einem Druckgefäß mit 5 bis 100 Mol Fluorwasserstoff und 0,5 + x bis 1,5 + x Mol Bortrifluorid, wobei x die Zahl der in der Ausgangsverbindung der genannten Formel (2) enthaltenen Sauerstoffatome bedeutet, versetzt, die Temperatur dieser Mischung auf -10° bis 100°C stellt und anschließend in diese vorgelegte Mischung Kohlenmonoxid einleitet bis ein Druck von 10 bis 150 bar erreicht ist und bei dem erreichten gewünschten Druck reagieren läßt.

2. Verfahren nach Anspruch 1, dadurch gekennzeichnet, daß man 20 bis 50 Mol Fluorwasserstoff einsetzt.

3. Verfahren nach mindestens einem der Ansprüche 1 und 2, dadurch gekennzeichnet, daß man mit 0,9 + x bis 1,2 + x, wobei x die in Anspruch 1 angegebene Bedeutung hat, Mol Bortrifluorid umsetzt.

4. Verfahren nach mindestens einem der Ansprüche 1 bis 3, dadurch gekennzeichnet, daß man 1 Mol Bortrifluorid einsetzt.

5. Verfahren nach mindestens einem der Ansprüche 1 bis 4, dadurch gekennzeichnet, daß man bei Temperaturen von 0° bis 80°C umsetzt.

6. Verfahren nach mindestens einem der Ansprüche 1 bis 5, dadurch gekennzeichnet, daß man bei einem CO-Druck von 20 bis 100 bar umsetzt.

7. Verfahren nach mindestens einem der Ansprüche 1 bis 6, dadurch gekennzeichnet, daß man den eingestellten CO-Druck bei Abfallen während der Umsetzung nachreguliert.

8. Verfahren nach mindestens einem der Ansprüche 1 bis 7, dadurch gekennzeichnet, daß man die Verbindung der genannten allgemeinen Formel (2) gelöst in einem gegenüber den Reaktionsteilnehmern inerten organischen Lösungsmittel vorlegt.

9. Verfahren nach mindestens einem der Ansprüche 1 bis 8, dadurch gekennzeichnet, daß man die Verbindung der genannten allgemeinen Formel (2) gelöst in Methylenchlorid, Chloroform, Tetrachlorkohlenstoff, Dichlorethan, Hexan oder Heptan vorlegt.

10. Verfahren nach mindestens einem der Ansprüche 1 bis 9, dadurch gekennzeichnet, daß man zunächst den Fluorwasserstoff vorlegt und dazu die Verbindung der genannten Formel (2) und anschließend das Bortrifluorid zusetzt.

## Claims

1. A process for preparing hydroxybenzaldehydes of the formula (1) 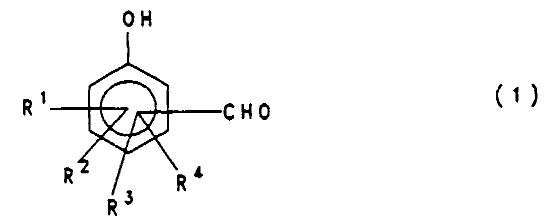 in which R¹, R², R³ and R⁴ are identical or different and are
hydrogen, fluorine, chlorine or bromine atoms, also alkyl(C₁-C₆) groups which may be substituted by fluorine atoms,
alkoxy(C₁-C₄) groups, phenyl or naphthyl groups which may be substituted at the aromatic nucleus by fluorine, chlorine or bromine atoms or by alkyl (C₁-C₄) or alkoxy (C₁-C₄) groups, also phenylalkyl (C₁-C₆) or naphthylalkyl (C₁-C₆) groups, which may be substituted at the aromatic nucleus by fluorine, chlorine or bromine atoms or by alkyl(C₁-C₄) or alkoxy(C₁-C₄) groups, also a cyclopentyl, cyclopentadienyl, cyclohexyl, cyclohexenyl or cyclohexadienyl group which may be substituted by alkyl (C₁-C₄) groups, also the phenoxy group which may be substituted by fluorine, chlorine or bromine atoms or by alkyl(C₁-C₄) or alkoxy(C₁-C₄) groups, and where the substituents R¹, R², R³ and R⁴ may with the hydroxybenzene ring carbon atoms on which they are located form one or two saturated or unsaturated isocyclic or heterocyclic rings which may be substituted by alkyl (C₁-C₄) groups, and in which the hydroxyl and aldehyde groups are in the ortho or para position with respect to one another, which comprises admixing 1 mol of a phenol of the formula (2) 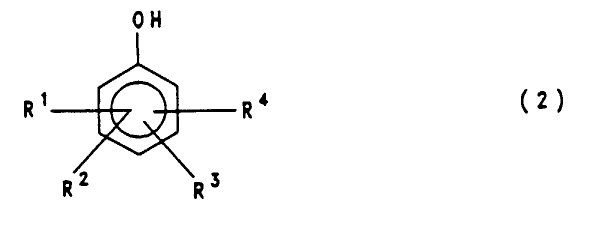 in which R¹, R², R³ and R⁴ have the meanings stated above, if desired in an organic solvent which is inert towards the reactants, in a pressure vessel with from 5 to 100 mol of hydrogen fluoride and from 0.5 + x to 1.5 + x mol of boron trifluoride, where x is the number of oxygen atoms contained in the starting compound of the stated formula (2), setting the temperature of this mixture to from -10° to 100°C and subsequently passing carbon monoxide into this initially charged mixture until a pressure of from 10 to 150 bar is reached and allowing the mixture to react at the desired pressure reached.

2. The process as claimed in claim 1, wherein from 20 to 50 mol of hydrogen fluoride are used.

3. The process as claimed in at least one of claims 1 and 2, wherein the reaction is carried out with from 0.9 + x to 1.2 + x, where x has the meaning given in claim 1, mol of boron trifluoride.

4. The process as claimed in at least one of claims 1 to 3, wherein 1 mol of boron trifluoride is used.

5. The process as claimed in at least one of claims 1 to 4, wherein the reaction is carried out at temperatures of from 0° to 80°C.

6. The process as claimed in at least one of claims 1 to 5, wherein the reaction is carried out at a CO pressure of from 20 to 100 bar.

7. The process as claimed in at least one of claims 1 to 6, wherein the set CO pressure is readjusted if it falls during the reaction.

8. The process as claimed in at least one of claims 1 to 7, wherein the initial charge comprises the compound of the stated formula (2) as a solution in an organic solvent which is inert towards the reactants.

9. The process as claimed in at least one of claims 1 to 8, wherein the initial charge comprises the compound of the stated formula (2) as a solution in methylene chloride, chloroform, carbon tetrachloride, dichloroethane, hexane or heptane.

10. The process as claimed in at least one of claims 1 to 9, wherein the order of addition is hydrogen fluoride, compound of the stated formula (2), boron trifluoride.

## Revendications

1. Procédé de préparation d'hydroxybenzaldéhydes de formule générale (1) 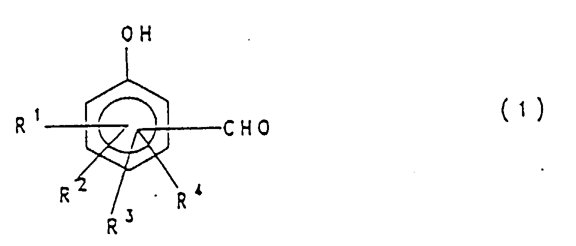 dans laquelle R¹, R², R³ et R⁴ sont identiques ou différents et représentent des atomes d'hydrogène, de fluor, de chlore ou de brome ; de plus des groupes alkyle en C₁-C₆ pouvant être substitués par des atomes de fluor, des groupes alcoxy en C₁-C₄, phényle ou naphtyle, qui peuvent être substitués sur le noyau aromatique par des atomes de fluor, de chlore ou de brome, par des groupes alkyle en C₁-C₄ ou des groupes alcoxy en C₁-C₄ de plus des groupes phényl(alkyle en C₁-C₆) ou naphtyl-(alkyle en C₁-C₆), qui peuvent être substitués sur le noyau aromatique par des atomes de fluor, de chlore ou de brome ou par des groupes alkyle en C₁-C₄ ou alcoxy en C₁-C₄ de plus un groupe cyclopentyle, cyclopentadiényle, cyclohexyle, cyclohexényle ou cyclohexadiényle, qui peuvent être substitués par des groupes alkyle en C₁-C₄ ; et de plus le groupe phénoxy, qui peut être substitué par des atomes de fluor, de chlore ou de brome, par des groupes alkyle en C₁-C₄ ou alcoxy en C₁-C₄, et où les substituants R¹, R², R³ et R⁴ peuvent, avec les atomes de carbone du noyau hydroxybenzénique sur lequel ils se trouvent, former un ou deux noyaux isocycliques ou hétérocycliques saturés ou insaturés, qui peuvent être substitués par des groupes alkyle en C₁-C₄ et dans lesquels les groupes hydroxyle et aldéhyde se trouvent en positionortho ou para l'un par rapport à l'autre, caractérisé en ce qu'on ajoute à 1 mole d'un phénol de formule générale (2) 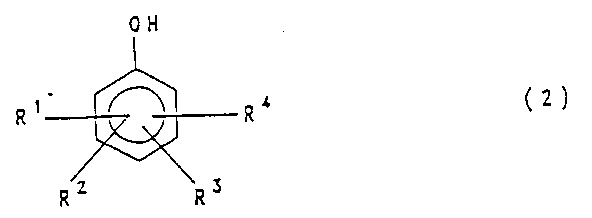 dans laquelle R¹, R², R³ et R⁴ ont les significations données ci-dessus, éventuellement dans un solvant organique inerte vis-à-vis des substances participant à la réaction, dans un récipient à pression, de 5 à 100 moles de fluorure d'hydrogène et de 0,5 + x à 1,5 + x de trifluorure de bore, où x est le nombre des atomes d'oxygène contenus dans le composé de départ ayant la formule (2) ci-dessus, on ajuste la température de ce mélange à une valeur de -10 à 100°C, puis on introduit dans ce mélange déjà en place du monoxyde de carbone jusqu'à arriver à une pression de 10 à 150 bar, et on fait réagir à la pression souhaitée atteinte.

2. Procédé selon la revendication 1, caractérisé en ce qu'on utilise de 20 à 50 moles de fluorure d'hydrogène.

3. Procédé selon au moins l'une des revendications 1 et 2, caractérisé en ce qu'on procède à la réaction avec 0,9 + x à 1,2 + x moles de trifluorure de bore, où x a la signification donnée dans la revendication 1.

4. Procédé selon au moins l'une des revendications 1 à 3, caractérisé en ce qu'on utilise 1 mole de trifluorure de bore.

5. Procédé selon au moins l'une des revendications 1 à 4, caractérisé en ce qu'on procède à la réaction à des températures de 0 à 80°C.

6. Procédé selon au moins l'une des revendications 1 à 5, caractérisé en ce qu'on procède à la réaction sous une pression de CO de 20 à 100 bar.

7. Procédé selon au moins l'une des revendications 1 à 6, caractérisé en ce qu'on réajuste pendant la réaction la pression ajustée de CO, quand cette dernière subit une diminution.

8. Procédé selon au moins l'une des revendications 1 à 7, caractérisé en ce qu'on part du composé ayant la formule générale (2) mentionnée ci-dessus, dissous dans un solvant organique inerte vis-à-vis des substances participant à la réaction.

9. Procédé selon au moins l'une des revendications 1 à 8, caractérisé en ce qu'on part du composé ayant la formule générale (2) ci-dessus, dissous dans du chlorure de méthylène, du chloroforme, du tétrachlorure de carbone, du dichloréthane, de l'hexane ou de l'heptane.

10. Procédé selon au moins l'une des revendications 1 à 9, caractérisé en ce qu'on met d'abord en place le fluorure d'hydrogène, et on y ajoute le composé ayant la formule (2) mentionnée ci-dessus, puis le trifluorure de bore.
